# FASCICULE DE BREVET EUROPEEN

(11) **EP 2 677 970 B1**
(45) Date de publication et mention de la délivrance du brevet: **22.04.2015**
(21) Numéro de dépôt: 12708902.7
(22) Date de dépôt: 10.02.2012
(51) Int. Cl.: A61F 2/44

(54) **PROTHESE DE DISQUE INTERVERTEBRAL**
BANDSCHEIBENPROTHESE
INTERVERTEBRAL-DISC PROSTHESIS

(30) Priorité: 21.02.2011 FR 1151393
(43) Date de publication de la demande: 01.01.2014
(73) Titulaire: Fournitures Hospitalieres Industrie, 29000 Quimper (FR)
(72) Inventeur: AARON, Alain, F-95470 Saint Witz (FR)
(74) Mandataire: Verriest, Philippe
(86) Numéro de dépôt international: PCT/FR2012/050296
(87) Numéro de publication internationale: WO 2012/114017

(56) Documents cités:
- WO-A1-2010/094881
- WO-A2-2006/055168
- US-A1- 2005 060 035
- US-A1- 2007 073 311

## Description

La présente invention concerne une prothèse destinée à remplacer un disque intervertébral endommagé de la colonne vertébrale.

La colonne vertébrale est constituée par un ensemble de vertèbres superposées reliées les unes aux autres par des disques fibro-cartilagineux, appelés disques intervertébraux. Ces disques intervertébraux ont un rôle fondamental dans la statique et la dynamique de la colonne vertébrale : ils assurent la mobilité des vertèbres entre elles.

Ces disques intervertébraux sont souvent l'objet de désordres relatifs à un tassement de vertèbres, une hernie discale, un déplacement de vertèbres ou encore une dégénérescence arthrosique intervertébrale. Ces désordres sont, bien souvent, la cause de douleurs ou de gènes fonctionnelles rebelles aux traitements médicaux ; dans certains cas, ils peuvent même être invalidants.

Le procédé utilisé pour soulager les patients atteints de ces désordres peut consister en une intervention chirurgicale visant à remplacer un disque endommagé par une prothèse de disque intervertébral.

L'implantation d'une telle prothèse de disque intervertébral peut être réalisée par des abords différents en fonction notamment de l'anatomie du patient. Il existe trois abords principaux pour les étages lombaires de L1/L2 à L5/S1, à savoir :
- l'abord antérieur rétropéritonéal possible en général pour tous les étages lombaires, mais pouvant parfois présenter des difficultés en raison de la présence de veines et artères,
- l'abord latéral possible pour tous les étages lombaires sauf L5/S1, qui est statistiquement l'un des étages nécessitant le plus souvent l'implantation d'une prothèse, en raison de la gêne occasionnée par la présence des ailes iliaques,
- l'abord antéro-latéral possible pour tous les étages lombaires, mais nécessitant un trajet de mise en place de la prothèse en oblique plus difficile.

Le document WO 2010/094881 décrit une prothèse de disque intervertébral comprenant des premier et deuxième plateaux, fixés chacun sur une des deux vertèbres adjacentes au disque vertébral à remplacer, entre lesquels est disposé un coussin de compression. Chaque plateau comporte, sur sa face intérieure, deux empreintes débouchant dans la face antérieure du plateau correspondant et disposées de part et d'autre du plan antéro-postérieur dudit plateau, les empreintes du premier plateau étant agencées pour être situées en regard des empreintes du deuxième plateau de manière à former deux cavités permettant l'accrochage d'un préhenseur-impacteur et l'implantation de la prothèse selon l'abord antérieur.

La prothèse décrite dans le document WO 2010/094881 ne permet pas une implantation selon l'abord latéral, et ne permet une implantation selon l'abord antéro-latéral que pour des chirurgiens très expérimentés. De ce fait, lorsque des veines et artères rendent complexes l'implantation selon l'abord antérieur, un chirurgien disposant d'une prothèse similaire à celle décrite dans le document WO 2010/094881 est souvent contraint à implanter, à la place de cette prothèse de disque intervertébral, une arthrodèse intervertébrale par l'abord antérieur. L'implantation d'une telle arthrodèse implique la fusion des deux vertèbres adjacentes au disque endommagé. Ce procédé a pour principal inconvénient de supprimer toute mobilité entre les deux vertèbres adjacentes au disque endommagé, et donc de concentrer les contraintes mécaniques sur les disques intervertébraux adjacents, ce qui peut entraîner un risque de détérioration de leur surface articulaire.

Afin d'éviter l'implantation d'une telle arthrodèse lorsque l'abord antérieur n'est pas possible, il est connu de mettre à la disposition des chirurgiens une prothèse de disque intervertébral présentant des moyens d'accrochage agencés pour coopérer avec des moyens d'accrochage complémentaire d'un préhenseur-impacteur, les moyens d'accrochage ménagés sur la prothèse étant conçus de manière à permettre une saisie de la prothèse selon une direction latéro-latérale à l'aide d'un préhenseur-impacteur et donc l'implantation de la prothèse selon l'abord latéral.

Ainsi, afin de permettre à un chirurgien d'implanter sur un patient une prothèse de disque intervertébral selon différents abords, il est nécessaire de mettre à la disposition de ce dernier au moins deux gammes de prothèses, à savoir une première gamme de prothèses de différentes tailles adaptées pour une implantation selon l'abord antérieur et une deuxième gamme de prothèses de différentes tailles adaptées pour une implantation selon l'abord latéral, et également une pluralité d'ancillaires adaptés pour la pose desdites prothèses.

Ce nombre élevé de prothèses et d'ancillaires complique le stockage de ces dernières et augmente les coûts liés à celui-ci. En outre, ce nombre élevé de prothèses de disque complique la manutention de ces dernières par le personnel du bloc opératoire, et rend plus complexe le geste opératoire.

Il en résulte un nombre importants de prothèses et d'ancillaires à mettre à la disposition du chirurgien, ce qui complexifie le geste opératoire.

II doit être noté que le choix d'abord planifié en pré-opératoire par le chirurgien peut être modifié en per-opératoire, du fait de la présence de tissus rendant complexe la pose de la prothèse selon l'abord initialement choisi.

Dans ce cas de figure, une nouvelle prothèse doit être sélectionnée dans la gamme de prothèses adaptées pour l'implantation selon l'abord choisi par le chirurgien en per-opératoire, et la prothèse initialement sélectionnée doit être mise au rebut, ce qui augmente encore le temps opératoire et les coûts de l'intervention chirurgicale.

De plus, la sélection d'une nouvelle prothèse nécessite l'utilisation d'un nouvel ancillaire, ce qui augmente le nombre d'ancillaires à stériliser après l'intervention chirurgicale.

La présente invention vise à remédier à ces inconvénients.

Le problème technique à la base de l'invention consiste donc à fournir une prothèse de disque intervertébral qui soit d'une réalisation simple, d'une structure compacte, et qui présente une parfaite sécurité d'utilisation, tout en permettant de simplifier l'intervention chirurgicale.

A cet effet, la présente invention concerne une prothèse de disque intervertébral comportant des premier et second plateaux rigides comprenant chacun une face intérieure et une face extérieure destinée à être fixée sur une des deux vertèbres adjacentes au disque intervertébral à remplacer, et un coussin de compression disposé entre les premier et second plateaux et solidaire des faces intérieures des premier et second plateaux, caractérisée en ce que chaque plateau comporte des premiers moyens d'accrochage comportant deux portions d'accrochage disposées sensiblement de manière symétrique de part et d'autre du plan médian antéro-postérieur dudit plateau, des deuxièmes moyens d'accrochage comportant deux portions d'accrochage disposées sensiblement de manière symétrique de part et d'autre d'un premier plan sensiblement perpendiculaire à la face intérieure dudit plateau et incliné d'un angle compris entre 50° et 70° par rapport au plan médian antéro-postérieur dudit plateau, et des troisièmes moyens d'accrochage opposés aux deuxièmes moyens d'accrochage par rapport au plan médian antéro-postérieur et comportant deux portions d'accrochage disposées sensiblement de manière symétrique de part et d'autre d'un deuxième plan sensiblement perpendiculaire à la face intérieure dudit plateau et incliné d'un angle compris entre 85° et 95°, et de préférence d'environ 90°, par rapport au plan médian antéro-postérieur dudit plateau, en ce que les premiers, deuxièmes et troisièmes moyens d'accrochage ménagés sur le premier plateau sont agencés pour être situés respectivement en regard des premiers, deuxièmes et troisièmes moyens d'accrochage ménagés sur le second plateau, et en ce que les premiers, deuxièmes et troisièmes moyens d'accrochage des premier et second plateaux sont destinés à coopérer respectivement avec des moyens d'accrochage complémentaires montés sur un organe préhenseur.

La configuration des différents moyens d'accrochage de la prothèse selon l'invention permet une implantation aisée de cette dernière selon les trois abords précités, à savoir selon l'abord antérieur en faisant coopérer les premiers moyens d'accrochage des deux plateaux avec des moyens d'accrochage complémentaires d'un organe préhenseur, selon l'abord antéro-latéral en faisant coopérer les deuxièmes moyens d'accrochage des deux plateaux avec des moyens d'accrochage complémentaires d'un organe préhenseur, et selon l'abord antérieur-latéral en faisant coopérer les troisièmes moyens d'accrochage des deux plateaux avec des moyens d'accrochage complémentaires d'un organe préhenseur.

Ces dispositions assurent à des chirurgiens la possibilité d'implanter une prothèse de disque intervertébral selon les trois abords précités, en mettant à leur disposition qu'une seule gamme de prothèses. Il en résulte une diminution importante du nombre de prothèses à mettre à la disposition des chirurgiens, et donc une simplification du geste opératoire.

Ces dispositions permettent en outre à un chirurgien de modifier son choix d'abord en per-opératoire, tout en conservant la même prothèse, ce qui simplifie encore le geste opératoire et limite les coûts de l'intervention chirurgicale.

Ces dispositions permettent également le retrait d'une prothèse par le même abord que celui utilisé pour la pose ou par l'un des deux autres abords possibles.

De façon préférentielle, les premiers, deuxièmes et troisièmes moyens d'accrochage des premier et second plateaux présentent une géométrie et des dimensions sensiblement identiques. Ces dispositions permettent l'implantation d'une prothèse avec le même ancillaire quel que soit l'abord choisi.

De préférence, chaque plateau comporte une face antérieure convexe délimitée au moins en partie par une portion de surface cylindrique s'étendant sur un angle supérieur à 160° et présentant un rayon de courbure constant. La portion de surface cylindrique s'étend de préférence sur un angle compris entre 160° et 220°, et par exemple sur un angle d'environ 180°.

Avantageusement, l'axe de la portion de surface cylindrique de chaque plateau s'étend sensiblement perpendiculairement à la face intérieure dudit plateau et s'étend sensiblement dans le plan médian antéro-postérieur dudit plateau.

De préférence, chaque plateau présente une largeur maximale au niveau du plan médian antéro-postérieur dudit plateau. Il doit être noté que l'on entend par largeur d'un plateau, la dimension de ce plateau selon une direction antéro-postérieure, et par longueur d'un plateau, la dimension de ce plateau selon une direction latéro-latérale.

De façon avantageuse, pour chaque plateau, l'intersection entre le premier plan et le plan médian antéro-postérieur dudit plateau est sensiblement confondue avec l'axe de la portion de surface cylindrique dudit plateau.

Selon un mode de réalisation de l'invention, pour chaque plateau, l'intersection entre le deuxième plan et le plan médian antéro-postérieur dudit plateau est sensiblement confondue avec l'axe de la portion de surface cylindrique dudit plateau.

Selon un autre mode de réalisation de l'invention, pour chaque plateau, l'intersection entre le deuxième plan et le plan médian antéro-postérieur dudit plateau est décalée par rapport à l'intersection entre le premier plan et le plan médian antéro-postérieur dudit plateau. Selon ce mode de réalisation, pour chaque plateau, l'intersection entre le deuxième plan et le plan médian antéro-postérieur dudit plateau peut par exemple être distante de l'intersection entre le premier plan et le plan médian antéro-postérieur dudit plateau d'une distance correspondant sensiblement au dixième de la largeur maximale dudit plateau. Selon ce mode de réalisation, pour chaque plateau, l'intersection entre le deuxième plan et le plan médian antéro-postérieur dudit plateau peut par exemple être distante de la face antérieure dudit plateau d'une distance correspondant sensiblement à la moitié de la largeur maximale dudit plateau. Selon ce mode de réalisation, l'intersection entre le deuxième plan et le plan médian antéro-postérieur dudit plateau est avantageusement décalée par rapport l'axe de la portion de surface cylindrique dudit plateau.

Selon une caractéristique avantageuse de l'invention, le rayon de courbure de la portion de surface cylindrique de chaque plateau est sensiblement égal à la moitié de la longueur maximale dudit plateau.

De préférence, le rapport entre la largeur maximale et la longueur maximale de chaque plateau est compris entre 0,66 et 0,75.

Selon un mode de réalisation de l'invention, chaque portion d'accrochage est formée au moins en partie par un rebord d'accrochage délimité par une encoche ménagée dans le plateau correspondant et débouchant dans la périphérie dudit plateau.

De façon préférentielle, chaque encoche s'étend sur une portion de l'épaisseur du plateau correspondant et débouche dans la face intérieure dudit plateau. Ces dispositions permettent de conserver une surface d'appui optimisée de la prothèse sur les corps vertébraux osseux, et de constituer une zone qui assure le positionnement dans un plan du préhenseur impacteur.

Avantageusement, la face extérieure du premier plateau et la face extérieure du second plateau forment un angle compris entre 10° et 20°.

De préférence, le premier plateau comporte, dans sa zone centrale, une pièce tubulaire tournée vers le second plateau, et le second plateau comporte, dans sa zone centrale, un plot de section inférieure à celle de la pièce tubulaire, tourné vers le premier plateau et engagé dans la pièce tubulaire.

De toute façon l'invention sera bien comprise à l'aide de la description qui suit en référence au dessin schématique annexé représentant, à titre d'exemples non limitatifs, trois formes d'exécution de cette prothèse de disque intervertébral.
Figure 1 est une vue en perspective d'une prothèse de disque intervertébral selon un premier mode de réalisation de l'invention.
Figure 2 est une vue arrière des deux plateaux de la prothèse de la figure 1.
Figure 3 est une vue de côté de la prothèse de la prothèse de la figure 1.
Figure 4 est une vue schématique de dessus de l'un des plateaux de la prothèse de la figure 1.
Figure 5 est une vue de dessus du plateau de la figure 4 sur lequel est monté un organe préhenseur d'un premier type.
Figure 6 est une vue schématique de dessus de l'un des plateaux d'une prothèse de disque intervertébral selon un deuxième mode de réalisation de l'invention.
Figure 7 est une vue schématique de dessus de l'un des plateaux d'une prothèse de disque intervertébral selon un troisième mode de réalisation de l'invention sur lequel est monté un organe préhenseur d'un deuxième type.

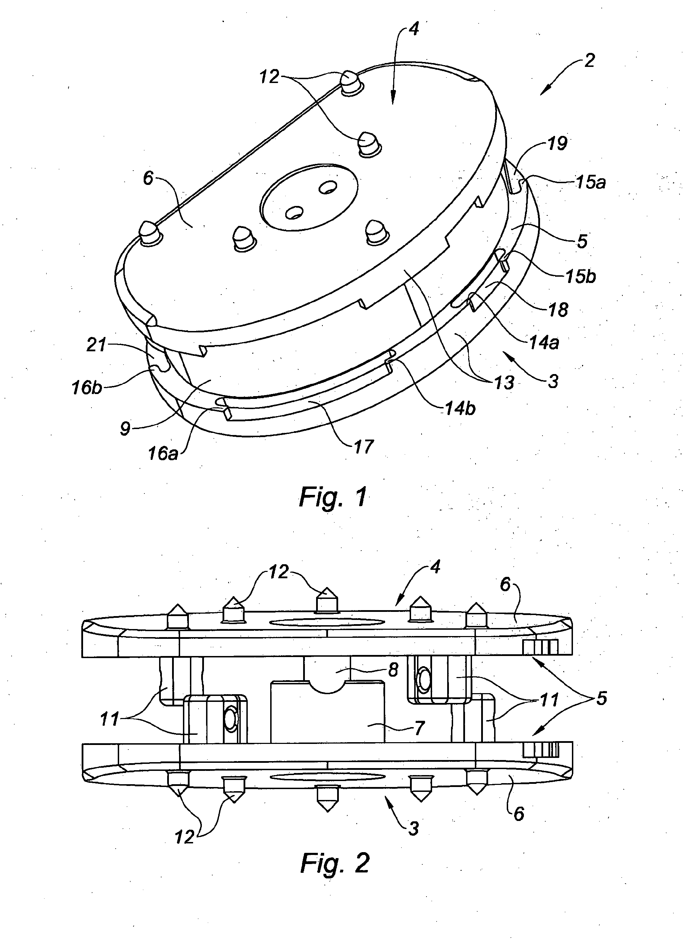

Les figures 1 à 5 représentent une prothèse de disque intervertébral lombaire 2 destinée à remplacer un disque intervertébral lombaire endommagé.

La prothèse 2 comporte un plateau inférieur 3 et un plateau supérieur 4. Les deux plateaux 3, 4 sont réalisés de préférence en alliage à base de titane.

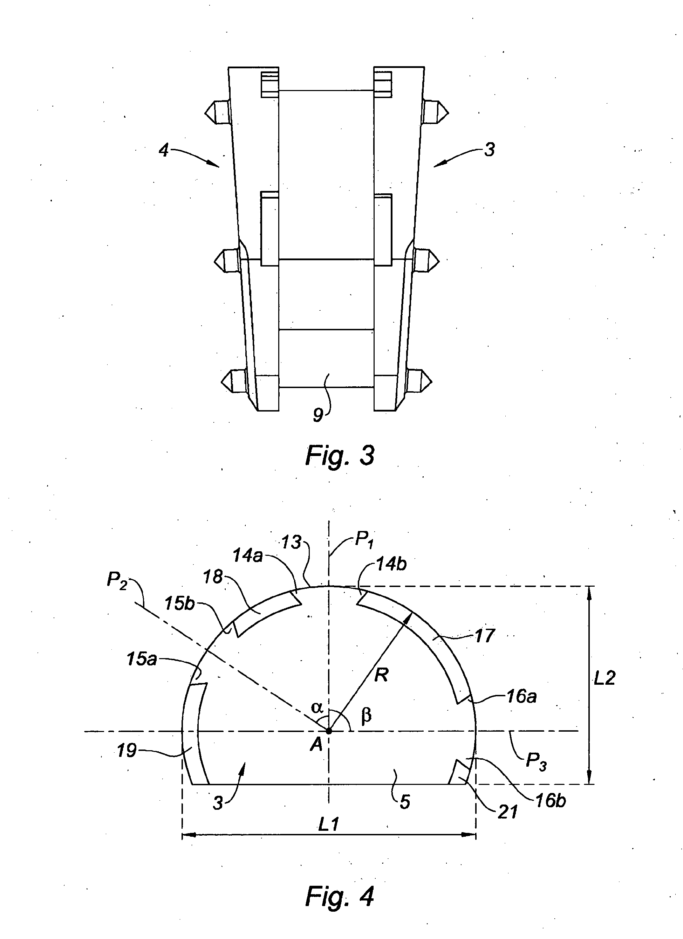

Chaque plateau 3, 4 comprend une face intérieure 5 et une face extérieure 6 sensiblement planes. La face extérieure 6 de chaque plateau 3, 4 est destinée à être fixée sur une des deux vertèbres lombaires adjacentes au disque intervertébral à remplacer. Comme montré plus particulièrement sur la figure 3, la face extérieure 6 du plateau inférieur et la face extérieure 6 du plateau supérieur forment de préférence un angle compris entre 10° et 20°.

Comme montré sur la figure 2, le plateau inférieur 3 comporte, dans sa zone centrale, une pièce tubulaire 7 de section circulaire tournée vers le plateau supérieur 4. Le plateau supérieur 4 comporte, dans sa zone centrale, un plot cylindrique ou tronconique 8 de section inférieure à celle de la pièce tubulaire 7, tourné vers le plateau inférieur 3 et engagé dans la pièce tubulaire 7. Il doit être noté que la somme des longueurs de la pièce tubulaire 7 et du plot 8 est supérieure à la distance entre les deux plateaux 3, 4.

La prothèse 2 comporte en outre un coussin de compression 9 disposé entre les plateaux inférieur et supérieur 3, 4, y compris dans le volume compris entre la pièce tubulaire 7 et le plot 8. Le coussin de compression 9 est solidaire des faces intérieures 5 des deux plateaux. Le coussin de compression 9 est réalisé avantageusement en un matériau compressible, de préférence de type polycarbonate uréthane.

Comme il est visible sur la figure 2, chaque plateau 3, 4 comporte, sur sa face intérieure 5, c'est-à-dire sur sa face tournée vers l'autre plateau, des ergots 11 favorisant l'accrochage du coussin de compression 9. En outre, chaque plateau 3, 4 comporte, sur sa face extérieure 6, des pointes de fixation 12 destinées à favoriser la fixation sur la vertèbre contre laquelle il est destiné à être en contact.

Chaque plateau 3, 4 comporte une face antérieure 13 convexe délimitée par une portion de surface cylindrique s'étendant sur un angle compris entre 180° et 220°, et présentant un rayon de courbure R constant. L'axe A de la portion de surface cylindrique de chaque plateau s'étend sensiblement perpendiculairement à la face intérieure 5 dudit plateau et s'étend sensiblement dans le plan médian antéro-postérieur P₁ dudit plateau. Le rayon de courbure R de la portion de surface cylindrique de chaque plateau est sensiblement égal à la moitié de la longueur maximale L₁ dudit plateau.

Le rapport entre la largeur maximale L₂ et la longueur maximale L₁ de chaque plateau est compris de préférence entre 0,66 et 0,75.

Chaque plateau comporte en outre des premiers, deuxièmes et troisièmes moyens d'accrochage agencés pour coopérer respectivement avec des moyens d'accrochage complémentaires montés sur un organe préhenseur. Les premiers, deuxièmes et troisièmes moyens d'accrochage ménagés sur le plateau inférieur sont agencés pour être situés respectivement en regard des premiers, deuxièmes et troisièmes moyens d'accrochage ménagés sur le plateau supérieur.

Les premiers moyens d'accrochage de chaque plateau comportent deux portions d'accrochage 14a, 14b disposées sensiblement de manière symétrique de part et d'autre du plan médian antéro-postérieur P₁ dudit plateau.

Les deuxièmes moyens d'accrochage de chaque plateau comportent deux portions d'accrochage 15a, 15b disposées sensiblement de manière symétrique de part et d'autre d'un premier plan P₂ sensiblement perpendiculaire à la face intérieure 5 dudit plateau et incliné d'un angle α compris entre 50° et 70° par rapport au plan médian antéro-postérieur P₁ dudit plateau. L'angle α est par exemple égal à environ 55° ou environ 60°.

Les troisièmes moyens d'accrochage de chaque plateau sont opposés aux deuxièmes moyens d'accrochage par rapport au plan médian antéro-postérieur P₁ et comportent deux portions d'accrochage 16a, 16b disposées sensiblement de manière symétrique de part et d'autre d'un deuxième plan P₃ sensiblement perpendiculaire à la face intérieure 5 dudit plateau et incliné d'un angle β compris entre 85° et 95°, et de préférence d'environ 90°, par rapport au plan médian antéro-postérieur P₁ dudit plateau.

Pour chaque plateau, l'intersection entre le premier plan P₂ et le plan médian antéro-postérieur P₁ dudit plateau et l'intersection entre le deuxième plan P₃ et le plan médian antéro-postérieur P₁ dudit plateau sont sensiblement confondues avec l'axe A de la portion de surface cylindrique dudit plateau.

Chaque plateau comprend en outre une pluralité d'encoches décalées angulairement les unes par rapport aux autres et débouchant dans la périphérie dudit plateau.

Comme montré sur les figures 1, 4 et 5, chaque plateau 3, 4 comprend plus particulièrement une première encoche 17 délimitant deux rebords d'accrochage formant respectivement les portions d'accrochage 14b, 16a, une deuxième encoche 18 délimitant deux rebords d'accrochage formant respectivement les portions d'accrochage 14a, 15b, une troisième encoche 19 délimitant un rebord d'accrochage formant la portion d'accrochage 15a, et enfin quatrième encoche 21 délimitant un rebord d'accrochage formant la portion d'accrochage 16b.

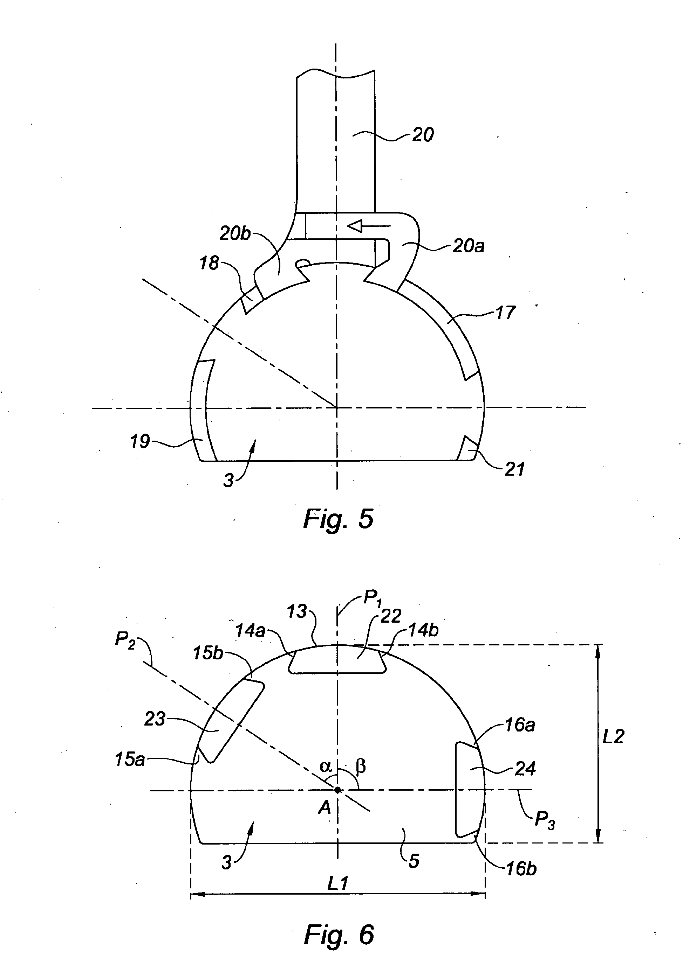

Chaque encoche s'étend avantageusement sur une portion de l'épaisseur du plateau correspondant et débouche dans la face intérieure 5 dudit plateau. Chaque encoche est de préférence conformée de telle sorte que le ou chaque rebord d'accrochage correspondant est délimité intérieurement une paroi intérieure concave, par exemple en forme de demi queue d'aronde (voir les figures 4 et 5) ou en forme partielle de cylindre (voir la figure 1).

Il est à noter que les différents moyens d'accrochage de la prothèse représentée sur les figures 1 à 5 sont agencés pour coopérer avec un organe préhenseur 20 qui assure la tenue de la prothèse par serrage de ses portions d'accrochage 20a, 20b, comme cela est plus particulièrement représenté sur la figure 5.

Il est également à noter que quel que soit l'abord sélectionné, donc quels que soient les moyens d'accrochage sélectionnés, les forces d'impactions exercées sur la prothèse par l'organe préhenseur sont exclusivement transmises aux plateaux, sans solliciter le coussin de compression 9.

Selon un mode de réalisation non représenté sur les figures, chaque plateau pourrait comporter six encoches décalées angulairement les unes par rapport aux autres, chaque encoche délimitant qu'un seul rebord d'accrochage.

Selon un deuxième mode de réalisation représenté sur la figure 6, chaque plateau comprend uniquement trois encoches décalées angulairement les unes par rapport aux autres. La première encoche 22 délimite les rebords d'accrochage formant les portions d'accrochage 14a, 14b appartenant aux premiers moyens d'accrochage, la deuxième encoche 23 délimite les rebords d'accrochage formant les portions d'accrochage 15a, 15b appartenant aux deuxièmes moyens d'accrochage, et la troisième encoche 24 délimite les rebords d'accrochage formant les portions d'accrochage 16a, 16b appartenant aux troisièmes moyens d'accrochage.

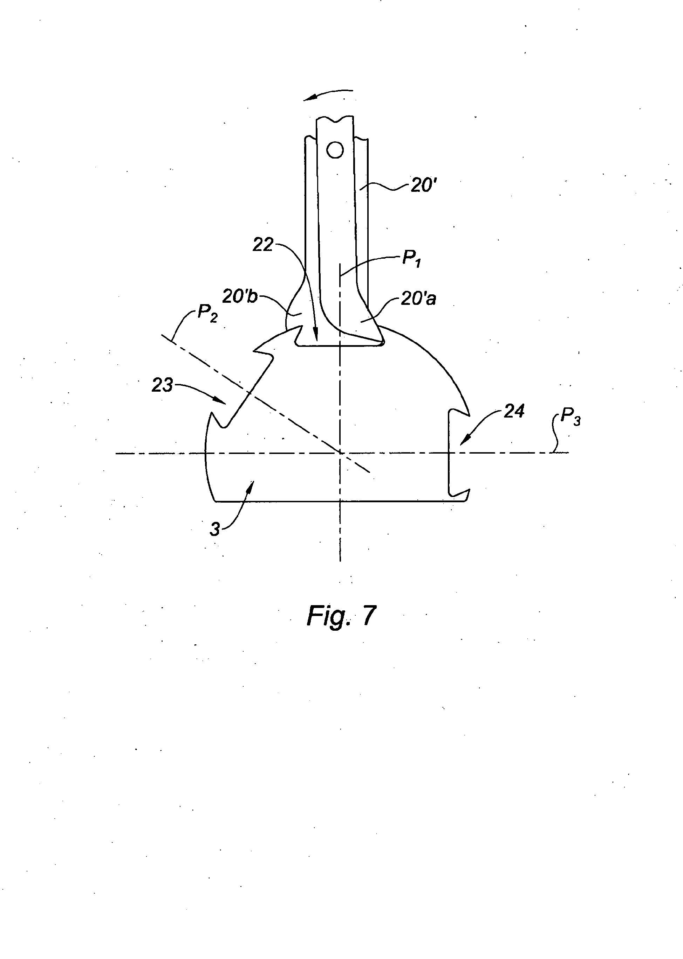

Il est à noter que les différents moyens d'accrochage de la prothèse représentée sur la figure 6 sont agencés pour coopérer avec un organe préhenseur 20' qui assure la tenue de la prothèse par écartement de ses portions d'accrochage 20'a, 20'b, comme cela est plus particulièrement représenté sur la figure 7.

Selon le mode de réalisation représenté sur la figure 7, chaque encoche est traversante et débouche respectivement dans les faces intérieure et extérieure dudit plateau.

Selon un mode de réalisation non représenté sur les figures, pour chaque plateau, l'intersection entre le deuxième plan et le plan médian antéro-postérieur dudit plateau est décalée par rapport à l'intersection entre le premier plan et le plan médian antéro-postérieur dudit plateau. Par exemple, l'intersection entre le deuxième plan P₃ et le plan médian antéro-postérieur P₁ dudit plateau est distante de la face antérieure 5 dudit plateau d'une distance correspondant sensiblement à la moitié de la largeur maximale L₂ dudit plateau.

Comme il va de soi, l'invention ne se limite pas aux seules formes d'exécution de cette prothèse de disque intervertébral, décrites ci-dessus à titre d'exemples, elle en embrasse au contraire toutes les variantes de réalisation.

## Revendications

1. Prothèse de disque intervertébral (2) comportant des premier et second plateaux (3, 4) rigides comprenant chacun une face intérieure (5) et une face extérieure (6) destinée à être fixée sur une des deux vertèbres adjacentes au disque intervertébral à remplacer, et un coussin de compression (9) disposé entre les premier et second plateaux et solidaire des faces intérieures des premier et second plateaux, **caractérisée en ce que** chaque plateau (3, 4) comporte des premiers moyens d'accrochage comportant deux portions d'accrochage (14a, 14b) disposées sensiblement de manière symétrique de part et d'autre du plan médian antéro-postérieur (P₁) dudit plateau, des deuxièmes moyens d'accrochage comportant deux portions d'accrochage (15a, 15b) disposées sensiblement de manière symétrique de part et d'autre d'un premier plan (P₂) sensiblement perpendiculaire à la face intérieure dudit plateau et incliné d'un angle (α) compris entre 50° et 70° par rapport au plan médian antéro-postérieur dudit plateau, et des troisièmes moyens d'accrochage opposés aux deuxièmes moyens d'accrochage par rapport au plan médian antéro-postérieur et comportant deux portions d'accrochage (16a, 16b) disposées sensiblement de manière symétrique de part et d'autre d'un deuxième plan (P₃) sensiblement perpendiculaire à la face intérieure dudit plateau et incliné d'un angle (β) compris entre 85° et 95°, et de préférence d'environ 90°, par rapport au plan médian antéro-postérieur dudit plateau, **en ce que** les premiers, deuxièmes et troisièmes moyens d'accrochage ménagés sur le premier plateau sont agencés pour être situés respectivement en regard des premiers, deuxièmes et troisièmes moyens d'accrochage ménagés sur le second plateau, **et en ce que** les premiers, deuxièmes et troisièmes moyens d'accrochage des premier et second plateaux sont destinés à coopérer respectivement avec des moyens d'accrochage complémentaires (20a, 20b) montés sur un organe préhenseur (20).

2. Prothèse de disque intervertébral selon la revendication 1, **caractérisée en ce que** chaque plateau (3, 4) comporte une face antérieure (13) convexe délimitée au moins en partie par une portion de surface cylindrique s'étendant sur un angle supérieur à 160° et présentant un rayon de courbure (R) constant.

3. Prothèse de disque intervertébral selon la revendication 2, **caractérisée en ce que** l'axe (A) de la portion de surface cylindrique de chaque plateau (3, 4) s'étend sensiblement perpendiculairement à la face intérieure (5) dudit plateau et s'étend sensiblement dans le plan médian antéro-postérieur (P₁) dudit plateau.

4. Prothèse de disque intervertébral selon la revendication 3, **caractérisée en ce que**, pour chaque plateau, l'intersection entre le premier plan (P₂) et le plan médian antéro-pôstérieur (P₁) dudit plateau est sensiblement confondue avec l'axe (A) de la portion de surface cylindrique dudit plateau.

5. Prothèse de disque intervertébral selon la revendication 3 ou 4, **caractérisée en ce que**, pour chaque plateau, l'intersection entre le deuxième plan (P3) et le plan médian antéro-postérieur (P1) dudit plateau est sensiblement confondue avec l'axe (A) de la portion de surface cylindrique dudit plateau.

6. Prothèse de disque intervertébral selon la revendication 3 ou 4, **caractérisée en ce que**, pour chaque plateau, l'intersection entre le deuxième plan (P3) et le plan médian antéro-postérieur (P1) dudit plateau est décalée par rapport à l'intersection entre le premier plan et le plan médian antéro-postérieur dudit plateau.

7. Prothèse de disque intervertébral selon l'une des revendications 2 à 6, **caractérisée en ce que** le rayon de courbure (R) de la portion de surface cylindrique de chaque plateau (3, 4) est sensiblement égal à la moitié de la longueur maximale (L1) dudit plateau.

8. Prothèse de disque intervertébral selon l'une des revendications 1 à 7, **caractérisée en ce que** le rapport entre la largeur maximale (L2) et la longueur maximale (L1) de chaque plateau est compris entre 0,66 et 0,75.

9. Prothèse de disque intervertébral selon l'une des revendications 1 à 8, **caractérisée en ce que** chaque portion d'accrochage (14a-16b) est formée au moins en partie par un rebord d'accrochage délimité par une encoche (17-24) ménagée dans le plateau correspondant et débouchant dans la périphérie dudit plateau.

10. Prothèse de disque intervertébral selon la revendication 9, **caractérisée en ce que** chaque encoche s'étend sur une portion de l'épaisseur du plateau correspondant et débouche dans la face intérieure (5) dudit plateau.

11. Prothèse de disque intervertébral selon l'une des revendications 1 à 10, **caractérisée en ce.que** la face extérieure (6) du premier plateau (3) et la face extérieure (6) du second plateau (4) forment un angle compris entre 10° et 20°.

## Patentansprüche

1. Bandscheibenprothese (2), umfassend erste und zweite starre Platten (3, 4), die jeweils eine innere (5) und eine äußere Seite (6) umfassen, die ausgelegt ist, um auf einem der zwei Wirbel, die der zu ersetzenden Bandscheibe benachbart sind, befestigt zu werden, und ein Kompressionskissen (9), das zwischen der ersten und zweiten Platte angeordnet und einstückig mit den inneren Seiten der ersten und zweiten Platte ist, **dadurch gekennzeichnet, dass** jede Platte (3, 4) erste Verankerungsmittel umfasst, die zwei Verankerungsabschnitte (14a, 14b) aufweisen, die im Wesentlichen auf symmetrische Weise auf beiden Seiten der mittleren antero-posterioren Ebene (P₁) der Platte angeordnet sind, zweite Verankerungsmittel, die zwei Verankerungsabschnitte (15a, 15b) aufweisen, die im Wesentlichen auf symmetrische Weise auf beiden Seiten einer ersten Ebene (P₂) angeordnet sind, die im Wesentlichen senkrecht zur inneren Seite der Platte und in einem Winkel (α) geneigt ist, der zwischen 50° und 70° mit Bezug auf die mittlere antero-posteriore Ebene der Platte liegt, und dritte Verankerungsmittel, die den zweiten Verankerungsmitteln mit Bezug auf die mittlere antero-posteriore Ebene gegenüberliegen, und zwei Verankerungsabschnitte (16a, 16b) aufweisen, die im Wesentlichen auf symmetrische Weise auf beiden Seiten einer zweiten Ebene (P₃) angeordnet sind, die im Wesentlichen senkrecht zur inneren Seite der Platte und in einem Winkel (β) geneigt ist, der zwischen 85° und 95° und vorzugsweise ungefähr 90° mit Bezug auf die mittlere antero-posteriore Ebene der Platte liegt, und dadurch, dass die ersten, zweiten und dritten Verankerungsmittel, die auf der ersten Platte angebracht sind, angeordnet sind, um sich jeweils gegenüber den ersten, den zweiten und den dritten Verankerungsmittel zu befinden, die auf der zweiten Platte angebracht sind, und dadurch, dass die ersten, zweiten und dritten Verankerungsmittel der ersten und zweiten Platte ausgelegt sind, um jeweils mit komplementären Verankerungsmitteln (20a, 20b) zusammenzuarbeiten, die auf einem Greiforgan (20) montiert sind.

2. Bandscheibenprothese nach Anspruch 1, **dadurch gekennzeichnet, dass** jede Platte (3, 4) eine konvexe vordere Seite (13) umfasst, die mindestens teilweise durch einen Abschnitt mit zylindrischer Oberfläche begrenzt ist, der sich auf einem Winkel von mehr als 160° erstreckt und einen konstanten Krümmungsradius (R) aufweist.

3. Bandscheibenprothese nach Anspruch 2, **dadurch gekennzeichnet, dass** sich die Achse (A) des Abschnitts mit zylindrischer Oberfläche jeder Platte (3, 4) im Wesentlichen senkrecht zur inneren Seite (5) der Platte erstreckt und sich im Wesentlichen auf der mittleren antero-posterioren (P₁) Ebene der Platte erstreckt.

4. Bandscheibenprothese nach Anspruch 3, **dadurch gekennzeichnet, dass** bei jeder Platte die Kreuzung zwischen der ersten Ebene (P₂) und der mittleren antero-posterioren Ebene (P₁) der Platte im Wesentlichen mit der Achse (A) des Abschnitts mit zylindrischer Oberfläche der Platte übereinstimmt.

5. Bandscheibenprothese nach Anspruch 3 oder 4, **dadurch gekennzeichnet, dass** bei jeder Platte die Kreuzung zwischen der zweiten Ebene (P₃) und der mittleren antero-posterioren Ebene (P₁) der Platte im Wesentlichen mit der Achse (A) des Abschnitts mit zylindrischer Oberfläche der Platte übereinstimmt.

6. Bandscheibenprothese nach Anspruch 3 oder 4, **dadurch gekennzeichnet, dass** bei jeder Platte die Kreuzung zwischen der zweiten Ebene (P₃) und der mittleren antero-posterioren Ebene (P₁) der Platte mit Bezug auf die Kreuzung zwischen der ersten Ebene und der mittleren antero-posterioren Ebene der Platte versetzt ist.

7. Bandscheibenprothese nach einem der Ansprüche 2 bis 6, **dadurch gekennzeichnet, dass** der Krümmungsradius (R) des Abschnitts mit zylindrischer Oberfläche jeder Platte (3, 4) im Wesentlichen gleich der Hälfte der maximalen Länge (L1) der Platte ist.

8. Bandscheibenprothese nach einem der Ansprüche 1 - 7, **dadurch gekennzeichnet, dass** die Beziehung zwischen der maximalen Breite (L2) und der maximalen Länge (L1) jeder Platte zwischen 0,66 und 0,75 liegt.

9. Bandscheibenprothese nach einem der Ansprüche 1 bis 8, **dadurch gekennzeichnet, dass** jeder Verankerungsabschnitt (14a - 16b) mindestens teilweise durch einen Verankerungsrand gebildet ist, der von einem Ansatzstück (17 - 24) begrenzt ist, das in der entsprechenden Platte angebracht ist und in den Umfang der Platte mündet.

10. Bandscheibenprothese nach Anspruch 9, **dadurch gekennzeichnet, dass** sich jedes Ansatzstück auf einem Abschnitt der Dicke der entsprechenden Platte erstreckt und in die innere Seite (5) der Platte mündet.

11. Bandscheibenprothese nach einem der Ansprüche 1 bis 10, **dadurch gekennzeichnet, dass** die äußere Seite (6) der ersten Platte (3) und die äußere Seite (6) der zweiten Platte (4) einen Winkel bilden, der zwischen 10° und 20° liegt.

## Claims

1. An intervertebral disc prosthesis (2) comprising first and second rigid plates (3, 4) each comprising an inner face (5) and an outer face (6) designed to be attached on one of the two vertebrae adjacent to the intervertebral disc to be replaced, and a compression pad (9) arranged between the first and second plates and secured to the inner faces of the first and second plates, **characterized in that** each plate (3, 4) comprises first attaching means including two attaching portions (14a, 14b) positioned substantially symmetrically on either side of the anteroposterior median plane (P₁) of said plate, second attaching means including two attaching portions (15a, 15b) positioned substantially symmetrically on either side of the first plane (P₂) substantially perpendicular to the interface of said plate and inclined by an angle (α) comprised between 50° and 70° relative to the anteroposterior median plane of said plate, and third attaching means opposite the second attaching means relative to the anteroposterior median plane and including two attaching portions (16a, 16b) positioned substantially symmetrically on either side of a second plane (P₃) substantially perpendicular to the inner face of said plate and inclined by an angle (β) comprised between 85° and 95°, preferably approximately 90°, relative to the anteroposterior median plane of said plate, **in that** the first, second and third attaching means formed on the first plate are arranged to be situated respectively across from the first, second and third attaching means formed on the second plate, and **in that** the first, second and third attaching means of the first and second plates are designed to cooperate respectively with complementary attaching means (20a, 20b) mounted on a gripping member (20).

2. The intervertebral disc prosthesis according to claim 1, **characterized in that** each plate (3, 4) includes a convex anterior face (13) at least partially delimited by a cylindrical surface portion extending over an angle greater than 160° and having a constant curve radius (R).

3. The intervertebral disc prosthesis according to claim 2, **characterized in that** the axis (A) of the cylindrical surface portion of each plate (3, 4) extends substantially perpendicular to the inner face (5) of said plate and extends substantially in the anteroposterior median plane (P₁) of said plate.

4. The intervertebral disc prosthesis according to claim 3, **characterized in that** for each plate, the intersection between the first plane (P₂) and the anteroposterior median plane (P₁) of said plate is substantially combined with the axis (A) of the cylindrical surface portion of said plate.

5. The intervertebral disc prosthesis according to claim 3 or 4, **characterized in that** for each plate, the intersection between the second plane (P3) and the anteroposterior median plane (P1) of said plate is substantially combined with the axis (A) of the cylindrical surface portion of said plate.

6. The intervertebral disc prosthesis according to claim 3 or 4, **characterized in that** for each plate, the intersection between the second plane (P3) and the anteroposterior median plane (P1) of said plate is offset relative to the intersection between the first plane and the anteroposterior median plane of said plate.

7. The intervertebral disc prosthesis according to one of claims 2 to 6, **characterized in that** the curve radius (R) of the cylindrical surface portion of each plate (3, 4) is substantially equal to half of the maximum length (L1) of said plate.

8. The intervertebral disc prosthesis according to one of claims 1 to 7, **characterized in that** the ratio between the maximum width (L2) and the maximum length (L1) of each plate is comprised between 0.66 and 0.75.

9. The intervertebral disc prosthesis according to one of claims 1 to 8, **characterized in that** each attaching portion (14a-16b) is formed at least partially by a attaching rim delimited by a notch (17-24) formed in the corresponding plate and emerging in the periphery of said plate.

10. The intervertebral disc prosthesis according to claim 9, **characterized in that** each notch extends over a portion of the thickness of the corresponding plate and emerges in the inner face (5) of said plate.

11. The intervertebral disc prosthesis according to one of claims 1 to 10, **characterized in that** the outer face (6) of the first plate (3) and the outer face (6) of the second plate (4) form an angle comprised between 10° and 20°.
